# EUROPEAN PATENT SPECIFICATION

(11) **EP 2 289 505 B1**
(45) Date of publication and mention of the grant of the patent: **06.03.2013**
(21) Application number: 10171504.3
(22) Date of filing: 30.07.2010
(51) Int. Cl.: A61K 31/19, A61K 31/70, A61K 31/7016, A61K 31/702, A61K 31/715, A23L 1/30, A61P 1/14

(54) **Nutritional composition and food supplement containing such a nutritional composition.**
Nährstoffzusammensetzung und die Nährstoffzusammensetzung enthaltender Lebensmittelzusatz
Composition nutritionnelle et complément alimentaire contenant ladite composition nutritionnelle

(30) Priority: 26.08.2009 IT CN20090008
(43) Date of publication of application: 02.03.2011
(73) Proprietor: Alpiflor S.R.L., 12026 Piasco (CN) (IT)
(72) Inventor: Scatolero, Daniele, 12026 Piasco (CN) (IT)
(74) Representative: Robba, Pierpaolo

(56) References cited:
- WO-A1-2004/026316
- DE-A1- 10 244 359
- JP-A- 2007 131 541
- STROWSKI M Z ET AL: "Probiotic carbohydrates reduce intestinal permeability and inflammation in metabolic diseases", GUT 2009 BMJ PUBLISHING GROUP GBR, vol. 58, no. 8, August 2009 (2009-08), pages 1044-1045, XP8119617,
- NILSSON ULF ET AL: "Bifidobacterium lactis Bb-12 and Lactobacillus salivarius UCC500 modify carboxylic acid formation in the hindgut of rats given pectin, inulin, and lactitol", JOURNAL OF NUTRITION, vol. 136, no. 8, August 2006 (2006-08), pages 2175-2180, XP8119610, ISSN: 0022-3166
- FRANCESCHI FRANCESCO ET AL: "Role of probiotics in patients with Helicobacter pylori infection", HELICOBACTER, vol. 12, no. Suppl. 2, November 2007 (2007-11), pages 59-63, XP002571863, ISSN: 1083-4389
- WILLIAMS L ET AL: "Dietary fiber and other alternative therapies and irritable bowel syndrome", TOPICS IN CLINICAL NUTRITION 2009 LIPPINCOTT WILLIAMS AND WILKINS USA, vol. 24, no. 3, July 2009 (2009-07), pages 262-271, XP002571864, ISSN: 0883-5691

## Description

### Field of the invention

The present invention relates to a nutritional composition; in particular the present invention relates to a nutritional composition which makes it possible to provide benefits to the intestine and may thus be used as an aid for alleviating intestinal disorders.

The present invention furthermore relates to a food supplement containing the above-stated composition, said food supplement making it possible to provide benefits to the intestine and thus being usable for specific medical purposes.

### Background of the invention

Intestinal disorders (intestinal irregularity, abdominal bloating and the like) are extremely widespread in today's society.

It is commonly thought, among other things, that such disorders may be aggravated by behavioural factors such as stress and poor diet which are currently extremely widespread. Intestinal disorders may furthermore be connected with nutritional deficiencies associated with diets or the after-effects of bacterial infections.

Finally, besides the above-stated disorders, there are recognised pathological conditions which affect the intestine, such as irritable bowel syndrome (IBS), which cause substantial abnormalities in gastrointestinal function.

It is now well established that the bacterial community resident in the gastrointestinal tract, the "intestinal bacterial flora", has a major impact on gastrointestinal function.

When treating disorders and pathological conditions of the gastrointestinal tract, it is accordingly known to use nutritional compositions which have a beneficial effect on the intestinal bacterial flora to assist any pharmacological treatments.

In particular, two strategies for maintaining a sound intestinal bacterial flora are known and generally used:
- administration of live microbial preparations, known as probiotics;
- intake of indigestible oligosaccharides, known as prebiotics, which have a stimulating action on the intestinal bacterial flora.

Both nutritional compositions separately containing probiotics and prebiotics and nutritional compositions which provide combined use thereof are known in the prior art; in this connection, combined probiotics and prebiotics are known as "symbiotics".

In practice, it is thought that the prebiotics provide an essential substrate for growth and proliferation of the probiotics.

By way of example, document WO 2004/026316 describes a composition containing prebiotics (in particular inulin), preferably administered in combination with probiotics, used to assist and promote proper fermentation in the colon.

If said nutritional compositions are to act properly, however, it is necessary for the probiotics to arrive intact in the intestine, preferably accompanied by a prebiotic support and, once there, to be correctly absorbed and to proliferate so that they can effectively perform their beneficial action on the intestinal bacterial flora.

Known compositions are incapable of ensuring that such a process takes place reliably.

The object of the present invention is to provide a nutritional composition and a food supplement containing such a composition which allow the disadvantages of the prior art to be overcome and which permit proper absorption and satisfactory proliferation of the probiotics in the intestine so as to ensure an effective beneficial action on the intestinal bacterial flora.

This and other objects are achieved by means of a nutritional composition and a food supplement as claimed in the appended claims.

### Description of the invention

The present invention provides a nutritional composition in which a probiotic is associated not only with an appropriate prebiotic support but also with butyric acid, or a salt thereof.

In the context of the present application, "probiotic" is in general taken to mean any live microbial nutritional supplement which has a favourable influence on the intestinal microbial equilibrium.

Microorganisms belonging to the genera *Streptococcus, Leuconostoc, Pediococcus, Propionibacterium*, *Bacillus, Bifidobacterium* and *Lactobacillus* may be used as probiotics. According to the invention, the probiotics used in the nutritional composition preferably belong to the genera *Bifidobacterium* and *Lactobacillus.*

Still more preferably, the probiotics used are *Bifidobacterium lactis* and *Bifidobacterium bifidus*.

In the context of the present application, "prebiotics" are in general taken to mean any fermentable oligosaccharides.

According to the invention, the prebiotics used in the nutritional composition are preferably selected from among fructooligosaccharides (FOS) and galactooligosaccharides (GOS).

Still more preferably, the prebiotics used are fructooligosaccharides.

For the purposes of the present invention, butyric acid or a salt thereof may be used in preparing the nutritional composition.

Preferably, either butyric acid or an organic or inorganic salt thereof is used.

Still more preferably, calcium butyrate is used.

The Applicant has surprisingly found, by means of laboratory investigations and tests carried out on a sample of patients suffering from irritable bowel syndrome, that the beneficial action of the probiotic on the bacterial flora of the intestine and in particular of the colon may be appreciably increased thanks to the presence of butyric acid, or a salt thereof.

This is because the butyric acid helps the probiotics to adhere to the epithelial tissue of the intestinal walls, so promoting absorption of the probiotics and proper bacterial colonisation of said intestinal walls.

The presence of butyric acid advantageously provides further beneficial effects:

Firstly, it has been found that butyric acid has an influence on the pH of the colon, causing it to fall and so bringing about a reduction in the growth of pathogenic bacteria (for example *Escherichia coli).* In this way, proliferation of the probiotics to the detriment of the pathogenic bacteria is considerably promoted and increased by the presence of butyric acid.

Furthermore, butyric acid has a positive effect on the healing mechanisms of the intestinal mucosa, so helping to maintain the structural and functional integrity of the mucosa. Finally, the combination of probiotics, prebiotics and butyric acid or a salt thereof has demonstrated an ability to promote proper functioning of the immune system.

The nutritional composition according to the invention thus makes it possible to provide the intestine, and in particular the colon, with all the components necessary for the correct nutrition thereof, so effectively contributing to the treatment of intestinal disorders.

In this respect, the nutritional composition according to the invention achieves the above-stated objects.

According to the invention, said nutritional composition comprises probiotics, prebiotics and butyric acid in any non-zero amount.

In any event, according to a preferred embodiment of the invention said composition comprises:
- from 0.05×10⁹ to 500×10¹¹ CFU of probiotics;
- from 0.10 to 50,000 mg of prebiotics;
- from 0.50 to 50,000 mg of butyric acid, or a salt thereof.

Still more preferably, said composition comprises:
- from 0.5×10⁹ to 2.5×10⁹ CFU of probiotics;
- from 50 to 150 mg of prebiotics;
- from 150 to 350 mg of butyric acid, or a salt thereof.

By way of an example, a nutritional composition according to the invention could comprise:
- 250 mg of butyric acid;
- 100 mg of prebiotics;
- 1.02×10⁹ CFU of probiotics.

The above-stated composition was used for a test on a sample of 50 patients of between 20 and 65 years of age (28 women and 22 men) suffering from irritable bowel syndrome. Among the patients treated, 20 had diarrhoea as the main symptom while 30 had constipation. Furthermore all the patients had flatulence as a symptom, 45 had abdominal pain, 40 changes in faecal consistency, 35 had food intolerance.

The patients were treated for two months with the composition according to the invention, at a first dose for the first month and at a second lower dose for the second month.

The test results, summarised in Table 1 below, demonstrate that the efficacy of the composition according to the invention in treating the symptoms of irritable bowel syndrome goes well beyond the results achievable with known compositions based on probiotics and/or prebiotics.

In particular, the composition according to the invention has shown itself to be effective in at least 80% of cases in providing relief to diarrhoea and constipation and has proven to be extraordinarily effective in reducing abdominal pain and changes in faecal consistency.

**Table 1 - Results from testing of 50 patients suffering from irritable bowel syndrome before and after taking the composition according to the invention for two months**

| Complaint reported by patients: | Before | After |
|---|---|---|
| Diarrhoea | 20 | 4 |
| Constipation | 30 | 5 |
| Flatulence | 50 | 10 |
| Changes in faecal density | 40 | 5 |
| Abdominal pain | 45 | 5 |
| Food intolerance | 35 | 10 |

The present invention furthermore provides a food supplement.

In the present context, the term "food supplement" should be understood in a broad sense as any ingestible food product capable of supplementing the user's normal diet, in particular to achieve specific benefits, in the present case specific benefits for the intestine and for the intestinal bacterial flora. For example, the category of "dietary foods for special medical purposes" explicitly provided in Italian and Community regulations (see Directive 1999/21/EC) falls within the definition of "food supplement" as understood in the present application.

The food supplement according to the invention, preferably in the form of a pill or tablet, contains a nutritional composition in which a probiotic is associated not only with an appropriate prebiotic support but also with butyric acid or a salt thereof. Said nutritional composition generally makes up the core of the food supplement, which is thus capable of providing benefits to the intestine and is thus usable for specific medical purposes.

Said food supplement furthermore comprises one or more external coatings which enable the nutritional composition to pass intact through the gastric tract, so that it may then be effectively released in the intestine.

Preferably, said one or more coatings furthermore enable the nutritional composition to pass through the intestine while remaining intact, so that it may be released only in the colon.

To this end, said one or more coatings are made from compounds which only dissolve at pH values of greater than 6.5.

According to a preferred embodiment, the food supplement according to the invention comprises a first coating which is impenetrable to gastric juices and to water. Said first coating may be made, for example, from shellac.

Again according to said preferred embodiment, the food supplement according to the invention comprises a second coating impenetrable to gastric juices and to bile acids. Said second coating may be made, for example, from modified starch.

The food supplement according to the invention may furthermore advantageously comprise additives which permit controlled dissolution of the food supplement and, consequently, controlled release of the nutritional composition.

According to a preferred embodiment of the invention, the food supplement contains polyvinylpyrrolidone (PVP) and hydroxypropylmethylcellulose (HPMC) as stabilising additives which delay the dissolution of the food supplement and extend the release of the nutritional composition, so ensuring release of probiotics, prebiotics and butyric acid along the whole length of the colon.

By way of example, a food supplement according to the invention could exhibit the following composition:

| | |
|---|---|
| Calcium butyrate | 0.3070000 |
| Prebiotic | 0.1000000 |
| Probiotic | 0.01100 |
| Shellac | 0.0100000 |
| Modified starch | 0.0150000 |
| PVP 30 | 0.0050000 |
| High density HPMC | 0.1890000 |
| Microcrystalline cellulose | 0.3118980 |
| Magnesium stearate | 0.0100000 |
| Citric acid | 0.0050000 |
| Colorant | 0.0000020 |
| Talc, titanium dioxide | 0.0150000 |

The food supplement of the above example can be prepared by the method described below:
- the ingredients listed in the above table are weighed out separately;
- the following ingredients are mixed for at least 15 minutes in a powder mixer: calcium butyrate, prebiotic, probiotic, PVP30, high density HPMC, microcrystalline cellulose;
- the magnesium stearate is added to the resultant mixture and mixing is continued for a further approx. 5 minutes;
- using an appropriate tabletting machine, the tablets which will make up the core of the finished product are prepared;
- the enteric film coating mixture is prepared, the shellac being weighed out and dissolved in absolute ethanol,
- the shellac is sprayed onto the tablets until the average weight of the tablets has increased by the amount specified in the formulation;
- the final film coating mixture is prepared by weighing out the modified starch, titanium dioxide, talc, colorant and citric acid and dissolving them all in water;
- the final film coating mixture is sprayed onto the coated tablet until the final weight is obtained.

The resultant food supplement has the following properties:

| | | |
|---|---|---|
| - | uniformity of mass: | ± 5%; |
| - | breaking load: | 98 Newton ± 3%; |
| - | moisture content: | <8%; |
| - | dimensions: | thickness max. 7 mm |
| | | length 19 mm |
| | | width 10 mm |
| - | losses due to friability: | <1% |
| - | disintegration time: | >10 h at pH >6.5 |
| - | total microbiological count | >100 CFU/g |
| - | yeasts and moulds | >10 CFU/g, |
| - | salmonellae | absent in 10 g |
| - | pathogens | absent. |

## Claims

1. A nutritional composition comprising any non-zero amount of:
- at least one probiotic, and
- at least one prebiotic,
**characterised in that** it furthermore comprises any non-zero amount of butyric acid or of a salt thereof.

2. A nutritional composition according to claim 1, wherein said at least one probiotic belongs to the genera *Bifidobacterium* or *Lactobacillus.*

3. A nutritional composition according to claim 1, wherein said at least one probiotic is *Bifidobacterium lactis* or *Bifidobacterium bifidus.*

4. A nutritional composition according to claim 1, wherein said at least one prebiotic belongs to the fructooligosaccharides (FOS) or galactooligasaccharides (GOS).

5. A nutritional composition according to claim 4, wherein said at least one prebiotic is a fructooligosaccharide.

6. A nutritional composition according to claim 1, wherein said composition comprises butyric acid itself.

7. A nutritional composition according to claim 1, wherein said composition comprises an organic or inorganic salt of butyric acid.

8. A nutritional composition according to claim 1, wherein said at least one probiotic, said at least one prebiotic and the butyric acid or said salt thereof are present in the following amounts
- from 0.05×10⁹ to 500×10¹¹ CFU of probiotic;
- from 0.10 to 50,000 mg of prebiotic;
- from 0.50 to 50,000 mg of butyric acid or of said salt thereof.

9. A nutritional composition according to claim 8, wherein said at least one probiotic, said at least one prebiotic and the butyric acid or said salt thereof are present in the following amounts
- from 0.5×10⁹ to 2.5×10⁹ CFU of probiotic;
- from 50 to 150 mg of prebiotic;
- from 150 to 350 mg of butyric acid or of said salt thereof.

10. A food supplement of the pill or tablet type, **characterised in that** it comprises a core composed of a composition according to any one of claims 1 to 9.

11. A food supplement according to claim 10 furthermore comprising one or more gastro-resistant coatings around said core.

12. A food supplement according to claim 11, wherein the compounds used to make said one or more gastro-resistant coatings are selected such that they only dissolve in the presence of a pH greater than 6.5.

13. A food supplement according to claim 11 or claim 12 comprising at least one first gastro-resistant coating of shellac and a second gastro-resistant coating of modified starch.

14. A food supplement according to claim 10, furthermore comprising at least one stabilising additive which permits controlled dissolution of said food supplement and controlled release of said nutritional composition.

15. A method for preparing a food supplement according to claim 12, at least comprising the steps of:
- providing in powder form said at least one probiotic, said at least one prebiotic and the butyric acid or said salt thereof;
- weighing out said at least one probiotic, said at least one prebiotic and the butyric acid or said salt thereof;
- mixing said at least one probiotic, said at least one prebiotic and the butyric acid or said salt thereof in a powder mixer for at least 15 minutes ;
- introducing the resultant mixture into a tabletting machine and compressing until a compact core is obtained;
- providing a gastro-resistant material capable of dissolving only in the presence of a pH greater than 6.5;
- dissolving said material in an appropriate solvent;
- spraying the resultant solution onto said compact core until complete coating is obtained.

## Patentansprüche

1. Nährstoffzusammensetzung, umfassend eine Nicht-Nullmenge von:
- mindestens einem Probiotikum, und
- mindestens einem Präbiotikum,
**dadurch gekennzeichnet, dass** sie ferner eine Nicht-Nullmenge von Buttersäure oder einem Salz davon umfasst.

2. Nährstoffzusammensetzung nach Anspruch 1, wobei dieses mindestens ein Probiotikum zu den Gattungen *Bifidobacterium* oder *Lactobacillus* gehöhrt.

3. Nährstoffzusammensetzung nach Anspruch 1, wobei dieses mindestens ein Probiotikum *Bifidobacterium lactis* oder *Bifidobacterium bifidus* ist.

4. Nährstoffzusammensetzung nach Anspruch 1, wobei dieses mindestens ein Präbiotikum zu den Frukto-Oligosacchariden (FOS) oder Galakto-Oligosacchariden (GOS) gehöhrt.

5. Nährstoffzusammensetzung nach Anspruch 4, wobei dieses mindestens ein Präbiotikum ein Frukto-Oligosaccharid ist.

6. Nährstoffzusammensetzung nach Anspruch 1, wobei diese Zusammensetzung Buttersäure selbst umfasst.

7. Nährstoffzusammensetzung nach Anspruch 1, wobei diese Zusammensetzung ein organisches oder anorganisches Salz der Buttersäure umfasst.

8. Nährstoffzusammensetzung nach Anspruch 1, wobei dieses mindestens ein Probiotikum, dieses mindestens ein Präbiotikum und die Buttersäure oder das Salz davon in den folgenden Mengen anwesend sind
- 0,05 x 10⁹ bis 500 x 10¹¹ KBE des Probiotikums;
- 0,10 bis 50000 mg des Präbiotikums;
- 0,50 bis 50000 mg der Buttersäure oder des Salzes davon.

9. Nährstoffzusammensetzung nach Anspruch 8, wobei dieses mindestens ein Probiotikum, dieses mindestens ein Präbiotikum und die Buttersäure oder das Salz davon in den folgenden Mengen anwesend sind
- 0,5 x 10⁹ bis 2,5 x 10⁹ KBE des Probiotikums;
- 50 bis 150 mg des Präbiotikums;
- 150 bis 350 mg der Buttersäure oder des Salzes davon.

10. Lebensmittelzusatz in der Form von Pillen oder Tabletten, **dadurch gekennzeichnet, dass** er einen Kern umfasst, der aus einer Zusammensetzung nach einem der Ansprüche 1 bis 9 besteht.

11. Lebensmittelzusatz nach Anspruch 10, ferner umfassend eine oder mehrere magensaftresistente Beschichtung(en) um den Kern herum.

12. Lebensmittelzusatz nach Anspruch 11, wobei die zur Herstellung dieser einen oder mehreren Beschichtung(en) eingesetzten Verbindungen so ausgewählt sind, dass sie sich nur in Anwesenheit eines pH-Wertes größer als 6,5 auflösen.

13. Lebensmittelzusatz nach Anspruch 11 oder 12, umfassend mindestens eine erste magensaftresistente Beschichtung aus Schellack und eine zweite Beschichtung aus modifizierter Stärke.

14. Lebensmittelzusatz nach Anspruch 10, ferner umfassend mindestens ein stabilisierendes Additiv, das eine kontrollierte Auflösung des Lebensmittelzusatzes und eine kontrollierte Freisetzung der Nährstoffzusammensetzung ermöglicht.

15. Verfahren zum Zubereiten eines Lebensmittelzusatzes nach Anspruch 12, mindestens umfassend die Schritte:
- Bereitstellen, in Pulverform, dieses mindestens einen Probiotikums, dieses mindestens einen Präbiotikums und der Buttersäure oder des Salzes davon;
- Wiegen dieses mindestens einen Probiotikums, dieses mindestens einen Präbiotikums und der Buttersäure oder des Salzes davon;
- Mischen dieses mindestens einen Probiotikums, dieses mindestens einen Präbiotikums und der Buttersäure oder des Salzes davon in einem Pulvermischer für mindestens 15 Minuten;
- Einführen der resultierenden Mischung in eine Tablettenmaschine und Pressen bis zum Erhalten eines kompakten Kerns;
- Bereitstellen eines magensaftresistenten Materials, das sich nur in Anwesenheit eines pH-Wertes größer als 6,5 auflösen kann;
- Auflösen dieses Materials in einem angemessenen Lösungsmittel;
- Sprühen der resultierenden Lösung auf den kompakten Kern bis zur Vervollständigung der Beschichtung.

## Revendications

1. Une composition nutritionnelle comprenant une quantité non nulle de:
- au moins un probiotique, et
- au moins un prébiotique,
**caractérisée en ce qu'**elle comprend en outre une quantité non nulle d'acide butyrique ou d'un de ses sels.

2. Une composition nutritionnelle selon la revendication 1, dans laquelle ledit au moins un probiotique appartient aux genres *Bifidobacterium* ou *Lactobacillus.*

3. Une composition nutritionnelle selon la revendication 1, dans laquelle ledit au moins un probiotique est *Bifidobacterium lactis* ou *Bifidobacterium bifidus.*

4. Une composition nutritionnelle selon la revendication 1, dans laquelle ledit au moins un prébiotique appartient aux fructo-oligosaccharides (FOS) ou aux galacto-oligosaccharides (GOS).

5. Une composition nutritionnelle selon la revendication 4, dans laquelle ledit au moins un prébiotique est un fructo-oligosaccharide.

6. Une composition nutritionnelle selon la revendication 1, dans laquelle ladite composition comprend de l'acide butyrique lui-même.

7. Une composition nutritionnelle selon la revendication 1, dans laquelle ladite composition comprend un sel organique ou inorganique d'acide butyrique.

8. Une composition nutritionnelle selon la revendication 1, dans laquelle ledit au moins un probiotique, ledit au moins un prébiotique et l'acide butyrique ou ledit au moins un de ses sels sont présents dans les quantités suivantes
- de 0,05 x 10⁹ à 500 x 10¹¹ UFC de probiotique;
- de 0,10 à 50.000 mg de prébiotique;
- de 0,50 à 50.000 mg d'acide butyrique ou dudit de ses sels.

9. Une composition nutritionnelle selon la revendication 8, dans laquelle ledit au moins un probiotique, ledit au moins un prébiotique et l'acide butyrique ou ledit au moins un de ses sels sont présents dans les quantités suivantes
- de 0,5 x 10⁹ à 2,5 x 10⁹ UFC de probiotique;
- de 50 à 150 mg de prébiotique;
- de 150 à 350 mg d'acide butyrique ou dudit de ses sels.

10. Un complément alimentaire de type pilule ou comprimé, **caractérisé en ce qu'**il comprend un noyau constituée d'une composition selon l'une quelconque des revendications 1 à 9.

11. Un complément alimentaire selon la revendication 10, comprenant en outre un ou plusieurs revêtements gastro-résistants autour dudit noyau.

12. Un complément alimentaire selon la revendication 11, dans lequel les composés utilisés pour fabriquer lesdits un ou plusieurs revêtements gastro-résistants sont choisis de telle sorte qu'ils ne se dissolvent qu'en présence d'un pH supérieur à 6,5.

13. Un complément alimentaire selon la revendication 11 ou la revendication 12, comprenant au moins un première revêtements gastro-résistant en gomme laque et un deuxième revêtement gastro-résistant en amidon modifié.

14. Un complément alimentaire selon la revendication 10, comprenant en outre au moins un additif de stabilisation qui permet une dissolution contrôlée dudit complément alimentaire et une libération contrôlée de ladite composition nutritionnelle.

15. Procédé de préparation d'un complément alimentaire selon la revendication 12, comprenant au moins les étapes consistant à:
- fournir sous forme de poudre ledit au moins un probiotique, ledit au moins un prébiotique et l'acide butyrique ou ledit au moins un de ses sels;
- peser ledit au moins un probiotique, ledit au moins un prébiotique et l'acide butyrique ou ledit au moins un de ses sels;
- mélanger ledit au moins un probiotique, ledit au moins un prébiotique et l'acide butyrique ou ledit au moins un de ses sels dans un mélangeur à poudre pendant au moins 15 minutes;
- introduire le mélange ainsi obtenu dans une machine de fabrication de comprimés et comprimer jusqu'à l'obtention d'un noyau compact;
- fournir un matériau gastro-résistant capable de se dissoudre seulement en présence d'un pH supérieur à 6,5;
- dissoudre ledit matériau dans un solvant approprié;
- pulvériser la solution résultante sur ledit noyau compact jusqu'à l'obtention d'un revêtement complet.
